# EUROPEAN PATENT SPECIFICATION

(11) **EP 2 634 260 B1**
(45) Date of publication and mention of the grant of the patent: **04.06.2014**
(21) Application number: 12750307.6
(22) Date of filing: 02.03.2012
(51) Int. Cl.: C12Q 1/04

(54) **BIOFILM-MARKING COMPOSITION AND METHOD FOR DETECTION OF SAME ON SURFACES**
BIOFILM-MARKIERUNGSZUSAMMENSETZUNG UND VERFAHREN ZU DEREN NACHWEIS AUF OBERFLÄCHEN
COMPOSITION DE MARQUAGE DE BIOFILMS ET PROCÉDÉ DE DÉTECTION DE CES DERNIERS SUR DES SURFACES

(30) Priority: 31.08.2011 ES 201131434 U
(43) Date of publication of application: 04.09.2013
(73) Proprietor: Betelgeux, S. L., 46701 Gandia Valencia (ES)
(72) Inventor: ORIHUEL IRANZO, Enrique, 46701 Gandia (Valencia) (ES); BERTO NAVARRO, Ramon, 46701 Gandia (Valencia) (ES); LORENZO CARTON, Fernando, 46701 Gandia (Valencia) (ES); LOPEZ TORMO, Celia, 46701 Gandia (Valencia) (ES); SAN JOSE SERRAN, Carmen, 46701 Gandia (Valencia) (ES); ORGAZ MARTIN, Belén, 46701 Gandia (Valencia) (ES)
(74) Representative: Ungria López, Javier
(86) International application number: PCT/ES2012/070133
(87) International publication number: WO 2013/030419

(56) References cited:
- WO-A1-2010/014715
- WO-A1-2010/070292
- WO-A1-2010/070292
- US-A- 5 741 662
- US-A1- 2006 263 843
- US-A1- 2008 044 811

## Description

### TECHNICAL FIELD OF THE INVENTION

This invention relates to the field of identification of biofilms, also known as biological films, biolayers or microbial films, formed by microorganisms, a technique linked to the chemical and biochemical analysis of substances and that has an important application in many areas: environment, food and agriculture and any other area in which growth of microorganisms occurs in respective devices and machinery used.

### BACKGROUND OF THE INVENTION

The products of the FilmTracer (Invitrogen, Ltd (United Kingdom)) family are specific dyes for cells in biofilms that allow monitoring the growth of biofilms on surfaces by means of fluorescence microscopy, after a contact period of 30 minutes. This method requires the use of a fluorescence microscope in order to observe the results, and does not allow *in situ* analysis of the surfaces, since it is necessary to transfer the sample up to the microscope.

On the other hand, Mathur et al. (Indian Journal of Medical Microbiology, 2006, 24(1):25-29) describe a method for the detection of the formation of staphilococos biofilms based on staining with Congo red. Nevertheless, this method requires sampling of the surface and subsequent incubation in the culture medium with the dye, and has been shown to produce false negatives.

The use of coloring agents for the detection of dental plaque (a form of biofilm) is already known, especially by means of the use of erythrosine or fluorescein (Gillings B., Harvey B., Journal of Dental Research, 1975, 54(3)). Nevertheless, these methods are not very specific and they require the use of special lamps for identification.

The most commonly known techniques in the field of identification and clearance of biofilms on surfaces require taking samples of said surface and their subsequent analysis in a microbiological laboratory. For example, enzymatic processes have been developed where the biofilm is detected, detached and eliminated through its exposure to a preparation containing enzymes (ES 2162593) or other biological markers such as proteins (WO 2009/018369 A2).

WO2010/070292 discloses compositions for staining biofilms comprising ionic surfactants. The photo-catalysts used for staining kill the biofilm bacteria.

WO2010/014715 discloses compositions that can comprise a wide variety of surfactants. Here the compositions are for preventing and reducing biofilms.

In the field of identification and characterization of biological samples, like biofilms and cells, compounds are also used for staining, although the use of these dyes always requires microscopy techniques to be used in conjunction.

In contrast to these methods, the present invention offers an advantageous alternative, being compositions for visual identification of biofilms on surfaces, as will be outlined below and also their method of application, which does not require sampling because they are applied in situ on the actual surface to be analyzed (non-destructive process); nor do they require microscopy analysis since they allow to detect the biofilm with the naked eye. Also, this method does not require specialized personnel or special equipment for its application, since it comes in a ready to use format and is also easy to transport.

### DESCRIPTION OF THE INVENTION

A first object of the present invention is a biofilms marking composition comprising:
- at least one staining agent in a weight percentage relative to the total weight of the composition of between 0.1% and 1% including both limit values;
- at least one non-ionic surfactant
in a percentage of between 0.1 % and 6.0%, including both limit values;
- at least one anionic surfactant in a percentage of between 2.0% and 8.0%, including both limit values, and
- water in a percentage of between 75.0% and 92.8%, including both limit values.

The above mentioned composition acts as visual marker of biofilms on surfaces, which allows their rapid and simple identification. The staining agent or agents, adhere in a specific manner to the biofilm matrix, and owing to the surfactants and chelating agents, the composition is resistant to washing with polar solvents (water, alcohol ...), in such a way that the biofilm is stained with a characteristic color and the staining agents do not run when cleaning or rinsing the surface to be analyzed with a polar solvent, thus the areas with biofilm remain marked with the coloring agent for its detection.

The main advantage of the present composition is that, in contrast to the habitual techniques for the detection of biofilms on surfaces, its use does not require taking samples from the surface to be analyzed (that is to say, it is a non-destructive technique), and hence renders subsequent microbiological analysis in laboratory unnecessary, because it is possible to verify visually on the surface in question, whether or not a biofilm has formed.

Diverse staining agents can be used, such as Rhodamine and its variants - such as Rhodamine B, Rhodamine 6G and Rhodamine 123 - Sudan Black B, Coomassie blue, green malachite, Sudan red, Congo Red, Basic Fuchsine, Erythrosine, ruthenium red and combinations thereof. The Rhodamine has an intense fuchsia coloring, staining the biofilm in this color.

The preferred staining agent is Rhodamine, and more preferably Rhodamine B. Rhodamine is capable of staining biofilms of different microorganisms, while other dyes are more specific or do not work so well on surfaces of different materials.

In one particular embodiment of the marking composition, the at least one non-ionic surfactant is present in a weight percentage of between 0.1 % and 6.0 %, including both limit values, with regard to the total weight of the composition. Preferably, the at least one non-ionic surfactant is selected from the group consisting of one amine, one amine oxide and one N-oxide. Other groups of non-ionic surfactants that can be used (they must generate foam) are: alkyl polyglucosides and ethoxylated fatty alcohols. More preferably, when the non-ionic surfactant is an amine oxide, the non-ionic surfactant is selected from the group consisting of laurylamine oxide and alkyldimethylamine oxide. Still more preferably, the amine oxide is N-oxide C10-C16-alkyldimethyl amine.

The composition may comprise, optionally, at least one chelating agent that improves the stability of the foam. The at least one chelating agent can be present in the composition in a weight percentage of between 0.1 % and 1.0 %, including both limit values, relative to the total weight of the composition. The chelating agent or agents can be any compound, well-known as such in the field, such as EDTA and NTA (already included), phosphonic acid derivatives such as phosphonates, and carboxylates (for example citric acid or gluconic acid derivatives). For example, the above mentioned agent is selected from the group consisting of amino trimethylene phosphonic acid, ethylenediamine tetra(methylenephosphonic acid), ethylenediamine tetraacetic acid, nitrilotriacetic acid and trisodium phosphate.

The anionic surfactant or surfactants are in a weight percentage of between 2.0 % and 8.0 %, including both limit values, relative to the total weight of the composition. Anionic surfactants might be, for example, carboxylic acids and salts thereof, sulphonic acids and salts thereof, esters of phosphoric acid and salts thereof. The above mentioned surfactants are preferably sulphuric acid esters, being selected more preferably from the group consisting of sodium lauryl ether sulphate, sodium alkyl ether sulphate and ammonium alkyl ether sulphate.

As for the water, the composition presents a percentage of between 75.0 % and 92.8 % including both limit values, hence this being the majority component.

In the invention, the biofilms marking composition presents the following composition:
- at least one staining agent in a weight percentage relative to the total weight of the composition of between 0.1% and 1%, including both limit values;
- at least one non-ionic surfactant, in a weight percentage relative to the total weight of the composition of between 0.1% and 6.0%, including both limit values;
- at least one anionic surfactant, in a weight percentage relative to the total weight of the composition of between 2.0% and 8.0% including both limit values; and
- water, in a weight percentage relative to the total weight of the composition of between 75.0% and 92.8% including both limit values.

In one particular additional embodiment of the invention, the biofilms marking composition presents the following composition:
- at least one staining agent, in a weight percentage relative to the total weight of the composition of between 0.1% and 1%, including both limit values;
- at least one non-ionic surfactant, in a weight percentage relative to the total weight of the composition of between 0.1% and 6.0%, including both limit values;
- at least one anionic surfactant, in a weight percentage relative to the total weight of the composition of between 2.0% and 8.0%, including both limit values;

- at least one chelating agent, in a weight percentage relative to the total weight of the composition of between 0.1% and 1.0%, including both limit values; and
- water, in a weight percentage relative to the total weight of the composition of between 75.0% and 92.8%, including both limit values.

Optionally, the marking composition may comprise one or several pH buffers in a weight percentage relative to the total weight of the composition of between 0 and 0.1 %, including both limit values. The above mentioned pH buffer or pH buffers is/are preferably selected from the group consisting of sodium hydroxide, potassium hydroxide and trisodium silicate.

In a more particular embodiment of the invention, which takes into account all the above-described variants of the marking composition in terms of its components, said composition presents the following formulation:
- at least one staining agent, in a weight percentage relative to the total weight of the composition of between 0.2% and 0.6%, including both limit values;
- at least one non-ionic surfactant, in a weight percentage relative to the total weight of the composition of between 2.0% and 3.5%, including both limit values;
- at least one anionic surfactant, in a weight percentage relative to the total weight of the composition of between 3.0% and 6.0%, including both limit values; and
- water, in a weight percentage relative to the total weight of the composition of between 82.9% and 89.5%, including both limit values.

Optionally, according to this embodiment, the composition may comprise at least one chelating agent, in a weight percentage relative to the total weight of the composition of between 0.3% and 0.5%, including both limit values;

When this marking composition comprises at least one pH buffer, the later is present in a weight percentage relative to the total weight of the composition of between 0.005% and 0.05%.

More preferably, the marking composition of biofilms on surfaces presents the following specific composition:
- Rhodamine B as staining agent, in a weight percentage relative to the total weight of the composition of 0.5%;
- N-oxide of C10-C16-alkyl dimethylamine as non-ionic surfactant, in a weight percentage relative to the total weight of the composition of 2.4%;
- sodium lauryl ether sulphate (LES) as anionic surfactant, in a weight percentage relative to the total weight of the composition of 4.3%;
- ethylenediamine tetraacetic acid (EDTA) as chelating agent, in a weight percentage relative to the total weight of the composition of 0.4%;
- water, in a weight percentage relative to the total weight of the composition of 92.3%;
- sodium hydroxide as pH buffer, in a weight percentage relative to the total weight of the composition of 0.01%.

Preferably, the marking composition of biofilms on surfaces, in any of the previous variants, is presented in the form of foam. Said foam, upon contact with biofilm, takes on the color of the staining agent. For example, in the case of Rhodamines, the foam that is generated has a fuchsia color.

A second object of the present invention is a spray, also called aerosol or vaporizer, which contains the biofilms marking composition hereby described, in any of its variants. Preferably, the above mentioned spray contains (and releases) the composition as a foam.

A third object of the invention is a method for visually identifying biofilms on surfaces by means of the described marking composition, in any of its variants, with said method being characterized in that it comprises at least the following stages:
- bringing the biofilms marking composition into contact with a surface to be analyzed, and
- rinsing the surface with a polar solvent.

As the staining agent of the marking composition is resistant to this rinsing step then, in case biofilms have developed on the surface, the areas where it is present will remain marked with the dye, allowing their rapid visual identification.

Preferably, the contact between the marking composition and the surface is carried out in one of the following ways: immersion of the surface in a bath of the marking composition, or pulverization of the composition on the surface by means of a spray or aerosol that contains the above mentioned composition. Preferably, the contact is carried out by means of pulverization on the surface by means of a spray or aerosol that contains the marking composition and that is released in the form of foam.

The composition can either be applied to the entire surface to be treated or to two or more areas of the same. In the latter case, the surface is not entirely covered but the composition is applied to two or more reference points that allow detecting where biofilm has formed, as well as its extension.

Preferably, the contact between composition and surface lasts between 1 and 20 minutes, including both limit values. More preferably, the contact time is between 2 and 10 minutes, and even more preferably between 3 and 15 minutes, including both limit values, and yet still more preferably for 5 minutes, a time after which the surface is rinsed with the polar solvent.

This polar solvent is preferably water or alcohol.

The composition of the present invention has its application field in work surfaces like tables, conveyor belts, cutting blades, hooks, trays, etc., as well as floors, walls and roofs. It is used for the hygiene control in the agricultural food, cosmetic, and chemical industries as well as in food outlets, hotels, etc.

### BRIEF DESCRIPTION OF THE FIGURES

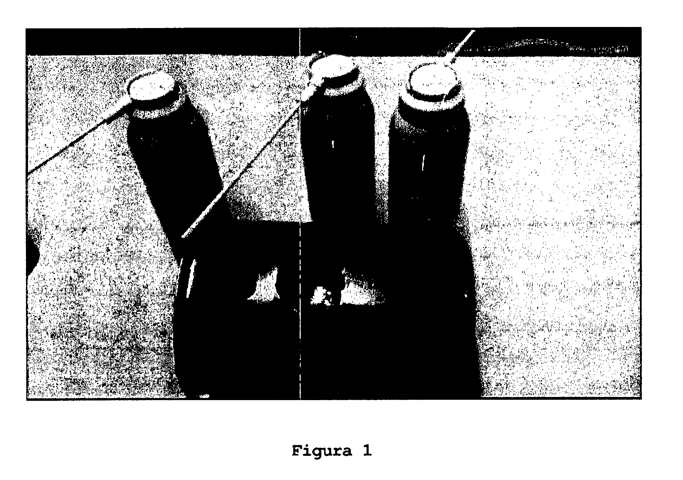

**Figure 1****.** It is a photograph of the surface of a metal vessel covered in three areas by the biofilms marking composition that is the object of the present invention. It is possible to visually check the existence of a biofilm on the surface of the vessel, thanks to the dye spray that contains the marking composition.

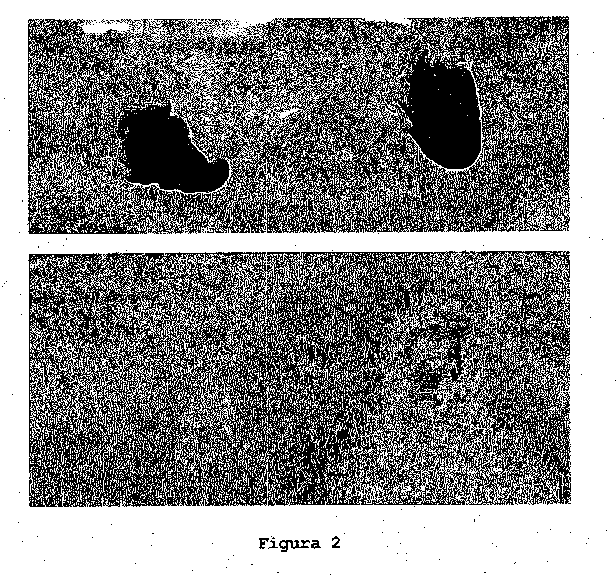

**Figure 2****.** It is a photograph of a metal surface covered in two areas by the biofilms marking composition that is the object of the present invention. In the area on the left, prior cleaning and disinfecting of the surface has been carried out in order to ensure an absence of biofilm, while the right area presents biofilms. The lower section of the photograph shows how the same surface looks after rinsing off the foam with polar solvent, and how the coloring is retained in the area that presents biofilms, allowing their visual identification.

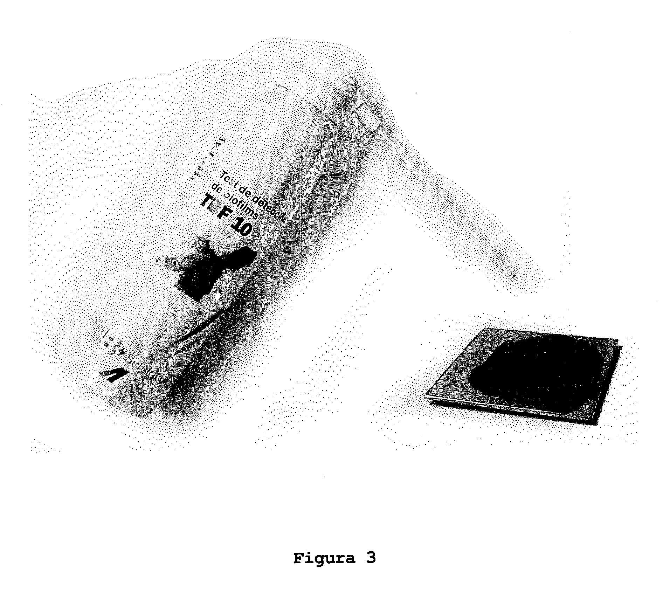

**Figure 3****.** It is a photograph of the container used for the application of the composition that is the object of the present invention in the form of dispensing foam spray. Also, the foam generated is shown on a metallic surface for the visual detection of biofilms.

### EMBODIMENTS OF THE INVENTION

What will now follow is a description, by way of non-limitative example, of a preferred embodiment of the invention, in which the preparation of a marking composition of biofilms on surfaces, according to the present invention, and its application, is presented in the form of a spray on a sample surface.

In order to perform the test, the following were added in a consecutive manner to a 2000 ml vessel subjected to constant agitation: 250 g of distilled water, 80 g of an amine oxide solution at 30 % (w/w), 10 g of EDTA 40 % (w/w), 160 g of sodium lauryl ether sulphate 27 % (w/w) and 500 grams of a rhodamine B solution at 1 %. The obtained mixture was filled into spray cans of 150 ml capacity, using 139 g of mixture per container, and introducing the propellant necessary for the generation of foam.

For the staining tests in the laboratory, *Pseudomonas fluorescens* ATCC 948 biofilms were caused to grow on glass panes immersed in a culture bath of this microorganism and incubated for 24 hours at 20°C. By means of the microbiological analysis of these panes, it could be verified that they presented microbial populations of approximately 10⁷ ufc/cm². Next, the panes were washed with water to detach the individual cells and a sufficient quantity of the composition was applied in the form of foam, enough to cover the entire surface of the panes. After an exposure time of contact of 10 minutes, the panes were rinsed with a water and ethanol solution, verifying that the panes on which biofilm had been made to grow were presenting the fuchsia color, characteristic of the composition, while a pane used as control (without growth of biofilm) was not presenting any traces of the dye.

Also, the same composition was tested in raw fish processing factory. For it, the composition was applied on a stainless steel sheet that is normally in contact with raw food, with said application being carried out after the habitual tasks of cleaning and disinfection. Additionally, one of the spots on which the composition was applied was previously scoured manually with a disinfectant so as to ensure the absence of microbial contamination. This test is shown in Figure 2.

After an exposure time of contact of 2 minutes, the tested surface was rinsed with water. It was observed that the area that had previously been manually scoured showed no traces of dye, while the area that had not been subjected to prior treatment was presenting clearly visibly remains of dye. As a cross-check, measurements of bioluminescence and microbial count were carried out in the area stained by the composition, with positive values of presence of enterobacteria and aerobic microorganisms being obtained there, as well as high values of bioluminescence of ATP. These results, as well as the presence of microbial contamination after cleaning and disinfection had been carried out, are indicative of the presence of biofilms on the surface, confirming the results obtained by means of the test carried out with the composition that is the object of this patent.

This way, the use of this composition to evaluate the presence of biofilms is much faster and simpler than the sampling of surfaces, incubation and microbial count that are usually employed for this purpose. Also, it is more specific than other techniques like the measurement of ATP bioluminescence, since the latter does not discriminate between live and dead or individual cells or components of a biofilm.

This method for the detection of biofilms also improves other biofilm staining methods, which have already been described that require surface examination by means of optical microscopy or by means of fluorescence microscopy, since these methods require specialized personnel, suitable equipment and a longer time of analysis.

## Claims

1. Biofilms visual marking composition, **characterized in that** it comprises:
- at least one staining agent in a weight percentage relative to the total weight of the composition of between 0.1% and 1%, including both limit values;
- at least one non-ionic surfactant in a percentage of between 0.1% and 6.0%, including both limit values,
- at least one anionic surfactant in a percentage of between 2.0% and 8.0%, including both limit values, and
- water in a percentage of between 75.0% and 92.8%, including both limit values.

2. Composition according to claim 1, wherein the at least one staining agent is Rhodamine.

3. Composition according to claim 2, wherein the at least one staining agent is selected from the group consisting of Rhodamine B, Rhodamine 6G and Rhodamine 123.

4. Composition according to claim 1 to 3, wherein the weight percentage relative to the total weight of the composition is as follows:
- the at least one non-ionic surfactant is present in a percentage of between 0.1% and 6.0%, including both limit values;
- the at least one anionic surfactant is present in a percentage of between 2.0% and 8.0%, including both limit values;
- at least one chelating agent is present in the composition in a percentage of between 0.1% and 1.0%, including both limit values;
- the water is present in a percentage between 75.0% and 92.8%, including both limit values.

5. Composition according to any one of claims 1 to 4, wherein the at least one non-ionic surfactant is selected from the group consisting of one amine, one amine oxide and one N-oxide.

6. Composition according to claim 5, wherein the at least one non-ionic surfactant is selected from the group consisting of lauryl amine oxide and alkyldimethylamine oxide when it is an amine oxide.

7. Composition according to any one of claims 1 to 6, wherein the at least one anionic surfactant is a sulphuric acid ester.

8. Composition according to claim 7, wherein the at least one anionic surfactant is selected from the group consisting of sodium lauryl ether sulphate, sodium alkyl ether sulphate and ammonium alkyl ether sulphate.

9. Composition according to any one of claims 4 to 8, wherein the chelating agent is selected from the group consisting of amino trimethylene phosphonic acid, ethylenediamine tetra(methylenephosphonic acid), ethylenediamine tetraacetic acid, nitrilotriacetic acid and trisodium phosphate.

10. Composition according to any one of claims 1 to 9, wherein the weight percentage relative to the total weight of the composition is as follows:
- at least one staining agent, at a percentage of between 0.2% and 0.6%, including both limit values;
- at least one non-ionic surfactant, at a percentage of between 2.0% and 3.5%, including both limit values;
- at least one anionic surfactant, at a percentage of between 3.0% and 6.0%, including both limit values;
- at least one chelating agent, at a percentage of between 0.3% and 0.5%, including both limit values; and
- water, at a percentage of between 82.9% and 89.5%, including both limit values.

11. Composition according to any one of claims 1 to 9, which comprises at least one pH buffer, in a weight percentage relative to the total weight of the composition of between 0.05% and 0.1%, including both limit values.

12. Composition according to claim 10 that comprises at least one pH buffer, in a weight percentage relative to the total weight of the composition of between 0.005% and 0.05%.

13. Composition according to any one of claims 11 or 12, wherein the at least one pH buffer is selected from the group consisting of sodium hydroxide, potassium hydroxide and trisodium silicate.

14. Composition according to any one of claims 1 to 13 that presents the following formulation:
- Rhodamine B as staining agent, in a weight percentage relative to the total weight of the composition of 0.5%;
- N-oxide of C10-C16-alkyldimethyl amine as non-ionic surfactant, in a weight percentage relative to the total weight of the composition of 2.4%;
- sodium lauryl ether sulphate (LES) as anionic surfactant, in a weight percentage relative to the total weight of the composition of 4.3%;
- ethylenediamine tetraacetic acid (EDTA) as chelating agent, in a weight percentage relative to the total weight of the composition of 0.4%;
- water, in a weight percentage relative to the total weight of the composition of 92.3%;
- sodium hydroxide as pH buffer, in a weight percentage relative to the total weight of the composition of 0.01%.

15. Marking spray for biofilms on surfaces, which contains the composition described in any one of the preceding claims.

16. Method for visually identifying biofilms on surfaces by means of the marking composition described in any one of claims 1 to 15, **characterized in that** it comprises at least the following stages:
- bringing the biofilms marking composition into contact with a surface to be analyzed, and
- rinsing the surface with a polar solvent.

17. Method according to the preceding claim, wherein the contact between the marking composition and the surface is carried out by means of pulverization of the composition on the surface with a spray that contains the marking composition.

18. Method according to the preceding claim, wherein the spray releases the composition in the form of foam.

19. Method according to any one of the preceding claims 16 to 18, wherein the contact between the composition and the surface lasts between 1 and 20 minutes, including both limit values.

20. Method according to the preceding claim, wherein the contact time is between 2 and 10 minutes, including both limit values.

21. Method according to any one of claims 16 to 20, wherein this polar solvent is water or alcohol.

## Patentansprüche

1. Zusammensetzung zur sichtbaren Markierung von Biofilmen, **dadurch gekennzeichnet, dass** sie umfasst:
- wenigstens ein Anfärbemittel in einem Gewichtsprozentanteil, bezogen auf das Gesamtgewicht der Zusammensetzung, zwischen 0,1 % und 1 %, einschließlich beider Grenzwerte;
- wenigstens einen nichtionischen oberflächenaktiven Stoff in einem Prozentanteil zwischen 0,1 % und 6,0 %, einschließlich beider Grenzwerte,
- wenigstens einen anionischen oberflächenaktiven Stoff in einem Prozentanteil zwischen 2,0 % und 8,0 %, einschließlich beider Grenzwerte, und
- Wasser in einem Prozentanteil zwischen 75,0 % und 92,8 %, einschließlich beider Grenzwerte.

2. Zusammensetzung nach Anspruch 1, wobei das wenigstens eine Anfärbemittel Rhodamin ist.

3. Zusammensetzung nach Anspruch 2, wobei das wenigstens eine Anfärbemittel ausgewählt ist aus der Gruppe bestehend aus Rhodamin B, Rhodamin 6G und Rhodamin 123.

4. Zusammensetzung nach Anspruch 1 bis 3, wobei der Gewichtsprozentanteil, bezogen auf das Gesamtgewicht der Zusammensetzung, wie folgt ist:
- der wenigstens eine nichtionische oberflächenaktive Stoff ist in einem Prozentanteil zwischen 0,1 % und 6,0 %, einschließlich beider Grenzwerte, vorhanden;
- der wenigstens eine anionische oberflächenaktive Stoff ist in einem Prozentanteil zwischen 2,0 % und 8,0 %, einschließlich beider Grenzwerte, vorhanden;
- wenigstens ein Chelatbildner ist in der Zusammensetzung in einem Prozentanteil zwischen 0,1 % und 1,0 %, einschließlich beider Grenzwerte, vorhanden;
- das Wasser ist in einem Prozentanteil zwischen 75,0 % und 92,8 %, einschließlich beider Grenzwerte, vorhanden.

5. Zusammensetzung nach einem der Ansprüche 1 bis 4, wobei der wenigstens eine nichtionische oberflächenaktive Stoff ausgewählt ist aus der Gruppe bestehend aus einem Amin, einem Aminoxid und einem N-Oxid.

6. Zusammensetzung nach Anspruch 5, wobei der wenigstens eine nichtionische oberflächenaktive Stoff ausgewählt ist aus der Gruppe bestehend aus Laurylaminoxid und Alkyldimethylaminoxid, wenn er ein Aminoxid ist.

7. Zusammensetzung nach einem der Ansprüche 1 bis 6, wobei der wenigstens eine anionische oberflächenaktive Stoff ein Schwefelsäureester ist.

8. Zusammensetzung nach Anspruch 7, wobei der wenigstens eine anionische oberflächenaktive Stoff ausgewählt ist aus der Gruppe bestehend aus Natriumlaurylethersulfat, Natriumalkylethersulfat und Ammoniumalkylethersulfat.

9. Zusammensetzung nach einem der Ansprüche 4 bis 8, wobei der Chelatbildner ausgewählt ist aus der Gruppe bestehend aus Aminotrimethylenphosphonsäure, Ethylendiamintetra(methylenphosphonsäure), Ethylendiamintetraessigsäure, Nitrilotriessigsäure und Trinatriumphosphat.

10. Zusammensetzung nach einem der Ansprüche 1 bis 9, wobei der Gewichtsprozentanteil, bezogen auf das Gesamtgewicht der Zusammensetzung, wie folgt ist:
- wenigstens ein Anfärbemittel, in einem Prozentanteil zwischen 0,2 % und 0,6 %, einschließlich beider Grenzwerte;
- wenigstens ein nichtionischer oberflächenaktiver Stoff, in einem Prozentanteil zwischen 2,0 % und 3,5 %, einschließlich beider Grenzwerte;
- wenigstens ein anionischer oberflächenaktiver Stoff, in einem Prozentanteil zwischen 3,0 % und 6,0 %, einschließlich beider Grenzwerte;
- wenigstens ein Chelatbildner, in einem Prozentanteil zwischen 0,3 % und 0,5 %, einschließlich beider Grenzwerte; und
- Wasser, in einem Prozentanteil zwischen 82,9 % und 89,5 %, einschließlich beider Grenzwerte.

11. Zusammensetzung nach einem der Ansprüche 1 bis 9, welche wenigstens einen pH-Puffer, in einem Gewichtsprozentanteil, bezogen auf das Gesamtgewicht der Zusammensetzung, zwischen 0,05 % und 0,1 %, einschließlich beider Grenzwerte, umfasst.

12. Zusammensetzung nach Anspruch 10, welche wenigstens einen pH-Puffer, in einem Gewichtsprozentanteil, bezogen auf das Gesamtgewicht der Zusammensetzung, zwischen 0,005 % und 0,05 %, umfasst.

13. Zusammensetzung nach einem der Ansprüche 11 oder 12, wobei der wenigstens eine pH-Puffer ausgewählt ist aus der Gruppe, bestehend aus Natriumhydroxid, Kaliumhydroxid und Trinatriumsilicat.

14. Zusammensetzung nach einem der Ansprüche 1 bis 13, welche die folgende Formulierung aufweist:
- Rhodamin B als Anfärbemittel, in einem Gewichtsprozentanteil, bezogen auf das Gesamtgewicht der Zusammensetzung, von 0,5 %;
- N-Oxid von C10-C16-Alkyldimethylamin als nichtionischer oberflächenaktiver Stoff, in einem Gewichtsprozentanteil, bezogen auf das Gesamtgewicht der Zusammensetzung, von 2,4 %;
- Natriumlaurylethersulfat (LES) als anionischer oberflächenaktiver Stoff, in einem Gewichtsprozentanteil, bezogen auf das Gesamtgewicht der Zusammensetzung, von 4,3 %;
- Ethylendiamintetraessigsäure (EDTA) als Chelatbildner, in einem Gewichtsprozentanteil, bezogen auf das Gesamtgewicht der Zusammensetzung, von 0,4 %;
- Wasser, in einem Gewichtsprozentanteil, bezogen auf das Gesamtgewicht der Zusammensetzung, von 92,3 %;
- Natriumhydroxid als pH-Puffer, in einem Gewichtsprozentanteil, bezogen auf das Gesamtgewicht der Zusammensetzung, von 0,01 %.

15. Markierungsspray für Biofilme auf Oberflächen, welches die in einem der vorangehenden Ansprüche beschriebene Zusammensetzung enthält.

16. Verfahren zum sichtbaren Identifizieren von Biofilmen auf Oberflächen mittels der Markierungszusammensetzung, die in einem der Ansprüche 1 bis 15 beschrieben ist, **dadurch gekennzeichnet, dass** es wenigstens die folgenden Stufen umfasst:
- Inkontaktbringen der Zusammensetzung zur Markierung von Biofilmen mit einer zu analysierenden Oberfläche, und
- Spülen der Oberfläche mit einem polaren Lösungsmittel.

17. Verfahren nach dem vorangehenden Anspruch, wobei der Kontakt zwischen der Markierungszusammensetzung und der Oberfläche mittels Zerstäuben der Zusammensetzung auf die Oberfläche mit einem Spray, welches die Markierungszusammensetzung enthält, durchgeführt wird.

18. Verfahren nach dem vorangehenden Anspruch, wobei das Spray die Zusammensetzung in Form eines Schaums freisetzt.

19. Verfahren nach einem der vorangehenden Ansprüche 16 bis 18, wobei der Kontakt zwischen der Zusammensetzung und der Oberfläche zwischen 1 und 20 Minuten, einschließlich beider Grenzwerte, dauert.

20. Verfahren nach dem vorangehenden Anspruch, wobei die Kontaktzeit zwischen 2 und 10 Minuten, einschließlich beider Grenzwerte, beträgt.

21. Verfahren nach einem der Ansprüche 16 bis 20, wobei dieses polare Lösungsmittel Wasser oder Alkohol ist.

## Revendications

1. Composition de marquage visuel de biofilms **caractérisée en ce qu'**elle comprend :
- au moins un agent colorant dans un pourcentage en poids par rapport au poids total de la composition entre 0,1 % et 1 %, incluant les deux valeurs limites ;
- au moins un tensioactif non ionique dans un pourcentage entre 0,1 % et 6,0 %, incluant les deux valeurs limites,
- au moins un tensioactif anionique dans un pourcentage entre 2,0 % et 8,0 %, incluant les deux valeurs limites,
et
- de l'eau dans un pourcentage entre 75,0 % et 92,8 %, incluant les deux valeurs limites.

2. Composition selon la revendication 1, où le au moins un agent colorant est la rhodamine.

3. Composition selon la revendication 2, où le au moins un agent colorant est choisi dans le groupe consistant en la rhodamine B, la rhodamine 6G et la rhodamine 123.

4. Composition selon les revendications 1 à 3, où le pourcentage en poids par rapport au poids total de la composition est le suivant :
- le au moins un tensioactif non ionique est présent dans un pourcentage entre 0,1 % et 6,0 %, incluant les deux valeurs limites ;
- le au moins un tensioactif anionique est présent dans un pourcentage entre 2,0 % et 8,0 %, incluant les deux valeurs limites ;
- au moins un agent chélatant est présent dans la composition dans un pourcentage entre 0,1 % et 1,0 %, incluant les deux valeurs limites ;
- l'eau est présente dans un pourcentage entre 75,0 % et 92,8 %, incluant les deux valeurs limites.

5. Composition selon l'une quelconque des revendications 1 à 4, où le au moins un tensioactif non ionique est choisi dans le groupe consistant en une amine, un oxyde d'amine et un N-oxyde.

6. Composition selon la revendication 5, où le au moins un tensioactif non ionique est choisi dans le groupe consistant en l'oxyde de laurylamine et un oxyde d'alkyldiméthylamine quand c'est un oxyde d'amine.

7. Composition selon l'une quelconque des revendications 1 à 6, où le au moins un tensioactif anionique est un ester d'acide sulfurique.

8. Composition selon la revendication 7, où le au moins un tensioactif anionique est choisi dans le groupe consistant en le lauryléther sulfate de sodium, un alkyléther sulfate de sodium et un alkyléther sulfate d'ammonium.

9. Composition selon l'une quelconque des revendications 4 à 8, où l'agent chélatant est choisi dans le groupe consistant en l'acide aminotriméthylènephosphonique, l'acide éthylènediaminetétra(méthylènephosphonique), l'acide éthylènediaminetétraacétique, l'acide nitrilotriacétique et le phosphate de trisodium.

10. Composition selon l'une quelconque des revendications 1 à 9, où le pourcentage en poids par rapport au poids total de la composition est le suivant :
- au moins un agent colorant, à un pourcentage entre 0,2 % et 0,6 %, incluant les deux valeurs limites ;
- au moins un tensioactif non ionique, à un pourcentage entre 2,0 % et 3,5 %, incluant les deux valeurs limites ;
- au moins un tensioactif anionique, à un pourcentage entre 3,0 % et 6,0 %, incluant les deux valeurs limites ;
- au moins un agent chélatant, à un pourcentage entre 0,3 % et 0,5 %, incluant les deux valeurs limites ;
et
- de l'eau, à un pourcentage entre 82,9 % et 89,5 %, incluant les deux valeurs limites.

11. Composition selon l'une quelconque des revendications 1 à 9, qui comprend au moins un tampon de pH, dans un pourcentage en poids par rapport au poids total de la composition entre 0,05 % et 0,1 %, incluant les deux valeurs limites.

12. Composition selon la revendication 10 qui comprend au moins un tampon de pH, dans un pourcentage en poids par rapport au poids total de la composition entre 0,005 % et 0,05 %.

13. Composition selon l'une quelconque des revendications 11 ou 12, où le au moins un tampon de pH est choisi dans le groupe consistant en l'hydroxyde de sodium, l'hydroxyde de potassium et le silicate de trisodium.

14. Composition selon l'une quelconque des revendications 1 à 13 qui présente la formulation suivante :
- la rhodamine B comme agent colorant, dans un pourcentage en poids par rapport au poids total de la composition de 0,5 % ;
- un N-oxyde de C10-C16-alkyldiméthylamine comme tensioactif non ionique, dans un pourcentage en poids par rapport au poids total de la composition de 2,4 % ;
- le lauryléther sulfate de sodium (LES) comme tensioactif anionique, dans un pourcentage en poids par rapport au poids total de la composition de 4,3 % ;
- l'acide éthylènediaminetétraacétique (EDTA) comme agent chélatant, dans un pourcentage en poids par rapport au poids total de la composition de 0,4 % ;
- de l'eau, dans un pourcentage en poids par rapport au poids total de la composition de 92,3 % ;
- de l'hydroxyde de sodium comme tampon de pH, dans un pourcentage en poids par rapport au poids total de la composition de 0,01 %.

15. Spray de marquage pour biofilms sur des surfaces, qui contient la composition décrite dans l'une quelconque des revendications précédentes.

16. Procédé pour identifier visuellement des biofilms sur des surfaces au moyen de la composition de marquage décrite dans l'une quelconque des revendications 1 à 15, **caractérisé en ce qu'**il comprend au moins les stades suivants :
- amener la composition de marquage de biofilms en contact avec une surface à analyser, et
- rincer la surface avec un solvant polaire.

17. Procédé selon la revendication précédente, où le contact entre la composition de marquage et la surface est accompli par pulvérisation de la composition sur la surface avec un spray qui contient la composition de marquage.

18. Procédé selon la revendication précédente, où le spray libère la composition sous forme de mousse.

19. Procédé selon l'une quelconque des revendications 16 à 18 précédentes, où le contact entre la composition et la surface dure entre 1 et 20 minutes, incluant les deux valeurs limites.

20. Procédé selon la revendication précédente, où la durée de contact est entre 2 et 10 minutes, incluant les deux valeurs limites.

21. Procédé selon l'une quelconque des revendications 16 à 20, où ce solvant polaire est l'eau ou un alcool.
